# EUROPEAN PATENT SPECIFICATION

(11) **EP 2 842 428 B1**
(45) Date of publication and mention of the grant of the patent: **08.08.2018**
(21) Application number: 13181912.0
(22) Date of filing: 27.08.2013
(51) Int. Cl.: A23L 27/20, A23G 3/48, A21D 2/08, A61K 8/33, A61K 8/97, A61K 31/12, A23G 4/06, A61K 31/164, A61Q 11/00

(54) **Oral composition**
Orale Zubereitung
Composition orale

(43) Date of publication of application: 04.03.2015
(73) Proprietor: Symrise AG, 37603 Holzminden (DE)
(72) Inventor: Van Houten, Michele, Tokyo, 106-0046 (JP); Sato, Megumi, Tokyo, 106-0046 (JP); Tatesawa, Katsumi, Saitama-ken, 343-0023 (JP); Ogino, Sawako, Kanagawa, 234-0055 (JP); Kawakami, Ken, Kanagawa-ken, 226-0027 (JP); Glaubitz, Gerald, 37671 Höxter (DE); Ikasari, Lilik, 609480 Singapore (SG)
(74) Representative: Eisenführ Speiser

(56) References cited:
- EP-A1- 2 716 303
- US-A1- 2008 242 740
- DATABASE FSTA [Online] INTERNATIONAL FOOD INFORMATION SERVICE (IFIS), FRANkFURT-MAIN, DE; 1993, TACHIYASHIKI K ET AL: "Effects of garnishes with dishes on human saliva secretion. Effects of kinome (sanshou, Japanese pepper leaf) and lemon peel on whole saliva secretion. (translated)", XP002717035, Database accession no. FS-1993-05-A-0070 & JOURNAL OF JAPANESE SOCIETY OF NUTRITION AND FOOD SCIENCE [NIHON EIYO SHOKURYO GAKKAI-SHI], vol. 45, no. 2, 1992, pages 123-128,
- DATABASE BIOSIS [Online] BIOSCIENCES INFORMATION SERVICE, PHILADELPHIA, PA, US; October 2005 (2005-10), SUGAI ETSUKO ET AL: "Quantitative analysis of sanshool compounds in Japanese pepper (Xanthoxylum piperitum DC.) and their pungent characteristics", XP002717036, Database accession no. PREV200600080960 & BIOSCIENCE BIOTECHNOLOGY AND BIOCHEMISTRY, vol. 69, no. 10, October 2005 (2005-10), pages 1958-1962, ISSN: 0916-8451
- SUGAI E ET AL: "Pungent qualities of sanshool-related compounds evaluated by a sensory test and activation of rat TRPV1", BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, vol. 69, no. 10, 1 January 2005 (2005-01-01), pages 1951-1957, XP002503936, ISSN: 0916-8451, DOI: 10.1271/BBB.69.1951

## Description

### FIELD OF INVENTION

The present invention belongs to the area of aromas and flavors and refers to an oral composition comprising hydroxy-alpha sanshool and 2-phenylbutenal and their use for inducing a salivating, moisturizing and/or watering effect in compositions designated for oral uptake.

### STATE OF THE ART

In the world of food articles aromas and flavors play important role for consumer acceptance and buying decisions.

Indeed, flavor is the sensory impression of a food or other substance, and is determined mainly by the chemical senses of taste and smell. The "trigeminal senses", which detect chemical irritants in the mouth and throat as well as temperature and texture, are also very important to the overall flavor perception. The flavor of food, as such, can be altered with natural or artificial aromas or flavors, which affect these senses. Although out of the three chemical senses, smell is the first and sometimes dominating impression, taste plays the most important role for consumer acceptance and buying decisions. Taste, however, is limited to seven basic impressions, namely sweet, sour, bitter, salty, umami (savoury), piquance and metallic.

Reference is made to the paper *"*Effects of garnishes with dishes on human saliva secretion", issued by TACHIYASHIKI ET AL [J. JPN. SOC. NUTR. FOOD SCI. Vol. 45 (2), S. 123-128 (1992**)]** describing that smell of Szechuan pepper (sanshou) increases human saliva secretion. In their paper titled *"*Quantitative analysis of sanshool compound in Japanese pepper" SUGAI ET AL show that the main constituents in the leaves of Szechuan pepper is alpha sanshool, while the fruits mainly contain hydroxy alpha sanshool **[**BIOSCI. BIOTECHNOL. BIOCHEM. VOL. 69(10), p 1958-1962 **(2005)].** The same authors provide experimental data for the threshold of certain sanshool derivatives in BIOSCI. BIOTECHNOL. BIOCHEM. VOL. 69(10), p 1951-1957 (2005**).**

US 2008/0242740 A1 (SYMRISE) relates to aroma compositions of (i) certain saliva-stimulating alkamides having a tingling, pungent and/or hot flavor (such as for example pellitorines, spilanthol) with (ii) hesperetin (5,7-dihydroxy-3-(3-hydroxy-4-methoxyphenyl)-chroman-4-one) or the enantiomers and/or salts thereof and/or (iii) 4-hydroxy-dihydrochalcones (3-(4-hydroxyphenyl)-1-phenylpropan-1-ones) and/or the salts thereof, the use thereof to enhance the sweet flavor of sweet-tasting substances or the sweet odor impression of aroma substances which give rise to a sweet odor impression, but in particular to enhance the sweet initial flavor or odor (initial sweetness). The invention thus relates to the use of said aroma compositions as general sweetness enhancers and enhancers of initial sweetness.

Finally, EP 2716303 A1 (TAKASAGO) concerns a stimulating agent comprising a pungent component having astringency, a stimulating composition including the agent, as well as a flavor or fragrance composition, a food, a drink, an oral care product and an external skin preparation containing the agent or the composition, and also relates to the use of a pungent component having astringency as a stimulating agent. Examples of the pungent component having astringency include a plant extract of *Asteraceae Spilanthes, Rutaceae Zanthoxyium, Rutaceae Fagara,* or *Piperaceae Piper,* or an amide derivative.

Although thousands of different aromas and flavors are obtainable from nature or organic synthesis providing nearly every sensory impression the consumer may think of there is still a need in the market for specific flavors or flavor cocktails that provide specific combinations of tastes and effects, such as for example watering effect at the beginning of the consumption followed by a savory taste somewhat later. This is a challenge that is typical especially for chewing gums and hard boiled candies. Also required are aromas or flavors that exhibit their sensual behavior at very low concentrations.

Therefore, the object of the present invention has been developing oral compositions comprising aroma or flavoring agents that provide at very low concentrations of less than 0.2 % b.w. simultaneously long lasting and strong salivating, moisturizing, and watering effects to said compositions.

### DESCRIPTION OF THE INVENTION

Object of the present invention is an oral composition comprising
(a) hydroxyl-alpha sanshool and
(b) 2-phenylbutenal, and optionally
(c) additional aroma or flavor compounds.

The invention is defined in the appended claims.

Surprisingly, it has been observed that the two component - taken alone or preferably in combination - provide at very low concentrations already a strong salivating, moisturizing and watering effect to oral compositions, preferably food compositions and even more preferred to chewing gums and (hard) candies. In particular a combination of both actives has been found very useful, since it provides these effects over a longer period than taken alone. It was also observed that the compounds - taken alone or in combination - show a strong tingling effect and were able to reduce bitterness of various other flavoring agents.

Preferably, the composition according to the invention contain a total amount of components (a+b) of about 0.0001 to about 0.1 % b.w., preferably about 0.001 to about 0.08 % b.w. and more preferred of about 0.01 to about 0.05 % b.w. The components (a) and (b) may be present in a ratio per weight of about 10:90 to about 90:10, preferably 25:75 to 75:25 and even more preferred of about 40:60 to about 60:40. Each of these alternatives may be combined disclosing a more preferred embodiment.

### HYDROXY-ALPHA-SANSHOOL

Hydroxy-alpha sanshool (I)¹ forming compound (a) is the key component of Sichuan pepper, in which it is present in amounts of about 3 % b.w.
¹IUPAC: (2E,6Z,8E,10E)-N-(2-hydroxy-2-methylpropyl)dodeca-2,6,8,10-tetraenamide

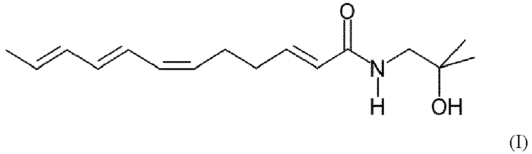

Sichuan pepper or Szechuan pepper, a common spice used in Asian cuisine, is derived from at least two species of the global genus *Zanthoxylum,* including *Z*. *simulans* and *Z*. *bungeanum.* The botanical name of the genus is composed of two Greek words that together mean "yellow wood" (referring to the brightly coloured sapwood possessed by several of the species). The genus belongs in the rue or citrus family, and, despite its name, is not closely related to either black pepper or chili pepper. The husk or hull (pericarp) around the seeds may be used whole, especially in Szechuan cuisine, and the finely ground powder is one of the blended ingredients for the five-spice powder. It is also used in traditional Chinese medicine. The pericarp (hull or husk) is the part that is most often used, but the leaves of various species are used as well in some regions of China.

Sichuan pepper has a unique aroma and flavor that is not hot or pungent like black, white or chili peppers. Instead, it has slight lemony overtones and creates a tingly numbness in the mouth that sets the stage for hot spices. According to Harold McGeein in "On Food and Cooking", 2nd edition, p 429

*"...they are not simply pungent; "they produce a strange, tingling, buzzing, numbing sensation that is something like the effect of carbonated drinks or of a mild electrical current (touching the terminals of a nine-volt battery to the tongue). Sanshools appear to act on several different kinds of nerve endings at once, induce sensitivity to touch and cold in nerves that are ordinarily non-sensitive, and so perhaps cause a kind of general neurological confusion. "*

The molecular mechanisms by which hydroxy-alpha sanshool induces these sensations have been a matter of debate. Although the compound is an agonist at the pain-integrating cation channels TRPV1 and TRPA1, newer evidence suggests that the two-pore domain potassium channels KCNK3, KCNK9, and KCNK18 are primarily responsible for hydroxy-alpha sanshool's effects. In traditional Japanese medicine the active is also known for improving male virility (e.g. JP 62 11675 A2).

In a first preferred embodiment of the present invention the sanshool is not used as a discrete compound, but as an extract of Sichuan pepper. These extracts may be prepared by methods known per se, i.e. for example by aqueous, alcoholic or aqueous/alcoholic extraction of the plants or parts thereof or shells of the litchi fruits. Suitable extraction processes are any conventional extraction processes, such as maceration, re-maceration, digestion, agitation maceration, vortex extraction, ultrasonic extraction, counter current extraction, percolation, re-percolation, evacolation (extraction under reduced pressure), diacolation and solid/liquid extraction under continuous reflux. Percolation is advantageous for industrial use. Litchi shells are preferably used as the starting material and may be mechanically size-reduced before the extraction process. Any size reduction methods known to the expert, for example freeze grinding, may be used. Preferred solvents for the extraction process are organic solvents, water (preferably hot water with a temperature above 80 °C and more particularly above 95 °C or mixtures of organic solvents and water, more particularly low molecular weight alcohols with more or less high water contents. Extraction with methanol, ethanol and water-containing mixtures thereof is particularly preferred. The extraction process is generally carried out at about 20 to about 100 °C and preferably at about 50 to about 70 °C. In one preferred embodiment, the extraction process is carried out in an inert gas atmosphere to avoid oxidation of the ingredients of the extract. This is particularly important where extraction is carried out at temperatures above 40 °C. The extraction times are selected by the expert in dependence upon the starting material, the extraction process, the extraction temperature and the ratio of solvent to raw material, etc. After the extraction process, the crude extracts obtained may optionally be subjected to other typical steps, such as for example purification, concentration and/or decoloration. If desired, the extracts thus prepared may be subjected, for example, to the selective removal of individual unwanted ingredients. The extraction process may be carried out to any degree, but is usually continued to exhaustion. Typical yields (=extract dry matter, based on the quantity of raw material used) in the extraction of the starting materials are of the order of about 1 to about 20, %, preferably about 2 to about 15 and more preferably about 5 to about 10 % b.w. - calculated on the starting materials.

Nevertheless, a particular preferred embodiment of the present invention refers to an extract that is obtained from a process using carbon dioxide as the extractant.

### 2-PHENYLBUTENAL

2-Phenylbutenal (II), forming compound (b), is obtainable according to the routine methods of organic chemistry.

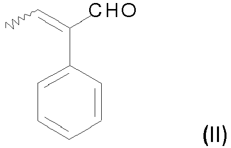

A suitable way for example is the condensation of phenyl acetaldehyde and acetaldehyde.

2-Phenylbutenal is also already known within various cosmetic compositions, for example in deodorants (WO 2010 146258 A) or as preservatives (WO 2008 149102 A). WO 2008 049581 A1 discloses the active as a component of the milk aroma. WO 2008 011742 A suggests it as a flavor precursor for food compositions. JP 2006 121958**,** JP 2006 025706**,** JP 2006020526**,** JP 2004135522 and DE 1921560 refer to 2-phenylbutenal for improving cacao, nut and coffee aromas.

### ADDITIONAL AROMA AND FLAVOR COMPOUNDS

Typically, flavors are added as a cocktail consisting of two, three or even up to ten different components. Thus, hydroxy-alpha sanshool and/or 2-phenylbutenal my combined with additional aroma and flavor compounds in a ratio of weight of for example (a+b):(c) equals 1:99 to 99:1, preferably about 10:90 to about 90:10, more preferably about 25:75 to about 75:25 and even more preferred about 40:6 to 60:40.

Suitable aroma compounds and flavoring agents (component c) are well known in the art and can be chosen from synthetic flavoring liquid and/or oils derived from plants leaves, flowers, fruits and so forth, and combinations thereof. Representative flavoring liquids include: artificial, natural or synthetic fruit flavors such as eucalyptus, lemon, orange, banana, grape, lime, apricot and grapefruit oils and fruit essences including apple, strawberry, cherry, orange, pineapple and so forth; bean and nut derived flavors such as coffee, cocoa, cola, peanut, almond and so forth; and root derived flavors such as licorice or ginger.

The flavoring agent is preferably selected from the group consisting of essential oils and extracts, tinctures and balsams, such as, for example, anisole, basil oil, bergamot oil, bitter almond oil, camphor oil, citronella oil, lemon oil; Eucalyptus citriodora oil, eucalyptus oil, fennel oil, grapefruit oil, camomile oil, spearmint oil, caraway oil, lime oil, mandarin oil, nutmeg oil (in particular nutmeg blossom oil = maces oil, mace oil), myrrh oil, clove oil, clove blossom oil, orange oil, oregano oil, parsley (seed) oil, peppermint oil, rosemary oil, sage oil (clary sage, Dalmatian or Spanish sage oil), star aniseed oil, thyme oil, vanilla extract, juniper oil (in particular juniper berry oil), wintergreen oil, cinnamon leaf oil; cinnamon bark oil, and fractions thereof, or constituents isolated therefrom.

It is of particular advantage if the oral composition according to the invention comprises at least one flavoring agent, preferably two, three, four, five, six, seven, eight or more flavoring agents chosen from the following group: menthol (preferably I-menthol and/or racemic menthol), anethole, anisole, anisaldehyde, anisyl alcohol, (racemic) neomenthol, eucalyptol (1,8-cineol), menthone (preferably L-menthone), isomenthone (preferably D-isomenthone), isopulegol, menthyl acetate (preferably L-menthyl acetate), menthyl propionate, carvone (preferably (-)-carvone, optionally as a constituent of a spearmint oil), methyl salicylate (optionally as a constituent of a wintergreen oil), eugenol acetate, isoeugenol methyl ether, beta-homocyclocitral, eugenol, isobutyraldehyde, 3-octanol, dimethyl sulfide, hexanol, hexanal, trans-2-hexenal, cis-3-hexenol, 4-terpineol, piperitone, linalool, 8-ocimenyl acetate, isoamyl alcohol, isovaleraldehyde, alpha-pinene, beta-pinene, limonene (preferably D-limonene, optionally as a constituent of an essential oil), piperitone, trans-sabinene hydrate, menthofuran, caryophyllene, germacrene D, cinnamaldehyde, mint lactone, thymol, gamma-octalactone, gamma-nonalactone, gamma-decalactone, (1,3E,5Z)-undecatriene, 2-butanone, ethyl formate, 3-octyl acetate, isoamyl isovalerate, cis- and trans-carvyl acetate, p-cymol, damascenone, damascone, cis-rose oxide, trans-rose oxide, fenchol, acetaldehyde diethyl acetal, 1-ethoxyethyl acetate, cis-4-heptenal, cis-jasmone, methyl dihydrojasmonate, 2'-hydroxypropiophenone, menthyl methyl ether, myrtenyl acetate, 2-phenylethyl alcohol, 2-phenylethyl isobutyrate, 2-phenylethyl isovalerate, geraniol, nerol and viridiflorol.

In particular preferred aroma or flavoring compounds encompass menthol, cineol, eugenol, thymol, cinnamic aldehyde, peppermint oil, spearmint oil, eucalyptus oil, thyme oil, cinnamon oil, clove oil, spruce needle oil, fennel oil, sage oil, aniseed oil, star anise oil, chamomile oil, and caraway oil, and their mixtures.

### ENCAPSULATION

The mixtures comprising compounds (a), (b) and optionally (c) may be encapsulated by means of a solid covering material, which is preferably selected from starches, degraded or chemically or physically modified starches (in particular dextrins and maltodextrins), gelatins, gum arabic, agar-agar, ghatti gum, gellan gum, modified and non-modified celluloses, pullulan, curdlan, carrageenans, alginic acid, alginates, pectin, inulin, xanthan gum and mixtures of two or more of said substances.

The solid covering material is preferably selected from gelatin (preferred are pork, beef, chicken and/or fish gelatins and mixtures thereof, preferably comprising at least one gelatin with a bloom value of greater than or equal to 200, preferably with a bloom value of greater than or equal to 240), maltodextrin (preferably obtained from maize (corn), wheat, tapioca or potato, preferred maltodextrins have a DE value of 10 - 20), modified cellulose (for example cellulose ether), alginates (for example Na-alginate), carrageenan (beta-, iota-, lambda- and/or kappa carrageenan), gum arabic, curdlan and/or agar-agar. Gelatin is preferably used, in particular, because of its good availability in different bloom values. Particularly preferred, especially for oral use are seamless gelatin or alginate capsules, the covering of which dissolves very rapidly in the mouth or bursts when chewing. Production may take place, for example, as described in EP 0389700 A1**,** US 4,251,195**,** US 6,214,376**,** WO 2003 055587 or WO 2004 050069 A1**.**

The capsules, however, may also represent micro-capsules. "Microcapsules" are understood to be spherical aggregates with a diameter of about 0.1 to about 5 mm which contain at least one solid or liquid core surrounded by at least one continuous membrane. More precisely, they are finely dispersed liquid or solid phases coated with film-forming polymers, in the production of which the polymers are deposited onto the material to be encapsulated after emulsification and coacervation or interfacial polymerization. In another process, liquid active principles are absorbed in a matrix ("microsponge") and, as microparticles, may be additionally coated with film-forming polymers. The microscopically small capsules, also known as nanocapsules, can be dried in the same way as powders. Besides single-core microcapsules, there are also multiple-core aggregates, also known as microspheres, which contain two or more cores distributed in the continuous membrane material. In addition, single-core or multiple-core microcapsules may be surrounded by an additional second, third etc. membrane. The membrane may consist of natural, semisynthetic or synthetic materials. Natural membrane materials are, for example, gum arabic, agar agar, agarose, maltodextrins, alginic acid and salts thereof, for example sodium or calcium alginate, fats and fatty acids, cetyl alcohol, collagen, chitosan, lecithins, gelatin, albumin, shellac, polysaccharides, such as starch or dextran, polypeptides, protein hydrolyzates, sucrose and waxes. Semisynthetic membrane materials are inter alia chemically modified celluloses, more particularly cellulose esters and ethers, for example cellulose acetate, ethyl cellulose, hydroxypropyl cellulose, hydroxypropyl methyl cellulose and carboxymethyl cellulose, and starch derivatives, more particularly starch ethers and esters. Synthetic membrane materials are, for example, polymers, such as polyacrylates, polyamides, polyvinyl alcohol or polyvinyl pyrrolidone.

Examples of known microcapsules are the following commercial products (the membrane material is shown in brackets) Hallcrest Microcapsules (gelatin, gum arabic), Coletica Thalaspheres (maritime collagen), Lipotec Millicapseln (alginic acid, agar agar), Induchem Unispheres (lactose, microcrystalline cellulose, hydroxypropylmethyl cellulose), Unicetin C30 (lactose, microcrystalline cellulose, hydroxypropylmethyl cellulose), Kobo Glycospheres (modified starch, fatty acid esters, phospholipids), Softspheres (modified agar agar) and Kuhs Probiol Nanospheres (phospholipids).

The active principles are released from the microcapsules by mechanical, thermal, chemical or enzymatic destruction of the membrane, normally during the use of the preparations containing the microcapsules. Despite the fact that the state of the art a huge range of possibilities for the encapsulation of actives, methods according to which a shell is obtained by coazervation, precipitation or polycondensation of anionic and cationic polymers has been quite suitable for the formation of stable capsules. Particularly, a preferred process for the encapsulation of active principles according to the present invention is characterised in that it comprises the steps of
(a) preparing a matrix from gel formers, cationic polymers and active principles;
(b) optionally dispersing said matrix in an oil phase; and
(c) treating said dispersed matrix with aqueous solutions of anionic polymers and optionally removing the in phase in the process.

Of course, anionic and cationic polymers in steps (a) and (c) can be exchanged.

**Gel formers.** In the context of the invention, preferred gel formers are substances which are capable of forming gels in aqueous solution at temperatures above 40° C. Typical examples of such gel formers are heteropolysaccharides and proteins. Preferred thermogelling heteropolysaccharides are agaroses which may be present in the form of the agar agar obtainable from red algae, even together with up to 30% by weight of non-gel-forming agaropectins. The principal constituent of agaroses are linear polysaccharides of Galactose and 3,6-anhydro-L-galactose with alternate 1,3- and 1,4-glycosidic bonds. The heteropolysaccharides preferably have a molecular weight of 110,000 to 160,000 and are both odourless and tasteless. Suitable alternatives are pectins, xanthans (including xanthan gum) and mixtures thereof. Other preferred types are those which in 1% by weight aqueous solution still form gels that do not melt below 80° C. and solidify again above 40° C. Examples from the group of thermogelling proteins are the various gelatines.

**Anionic polymers.** Salts of alginic acid are preferred for this purpose. The alginic acid is a mixture of carboxyl-containing polysaccharides with the following idealized monomer unit:

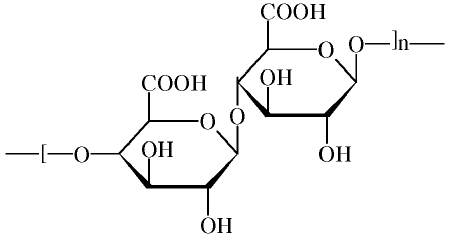

The average molecular weight of the alginic acid or the alginates is in the range from 150,000 to 250,000. Salts of alginic acid and complete and partial neutralization products thereof are understood In particular to be the alkali metal salts, preferably sodium alginate ("algin") and the ammonium and alkaline earth metal salts. Mixed alginates, for example sodium/magnesium or sodium/calcium alginates, are particularly preferred. In an alternative embodiment of the invention, however, carboxymethyl celluloses and anionic chitosan derivatives, for example the carboxylation and above all succinylation products are also suitable for this purpose.

**Cationic polymers.** Chitosans are biopolymers which belong to the group of hydrocolloids. Chemically, they are partly de-acetylated chitins differing in their molecular weights which contain the following - idealized - monomer unit:

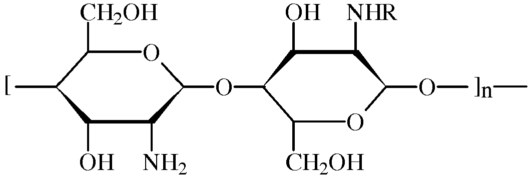

In contrast to most hydrocolloids, which are negatively charged at biological pH values, chitosans are cationic biopolymers under these conditions. The positively charged chitosans are capable of interacting with oppositely charged surfaces and are therefore used in cosmetic hair-care and body-care products and pharmaceutical preparations.

In a preferred embodiment of the invention a 1 to 10 and preferably 2 to 5% by weight aqueous solution of the gel former, preferably agar agar, is normally prepared and heated under reflux. A second aqueous solution containing the cationic polymer, preferably chitosan, in quantities of 0.1 to 2 and preferably 0.25 to 0.5% by weight and the active principle in quantities of 0.1 to 25 and preferably 0.25 to 10% by weight is added in the boiling heat, preferably at 80 to 100 ° C.; this mixture is called the matrix. Accordingly, the charging of the microcapsules with active principles may also comprise 0.1 to 25% by weight, based on the weight of the capsules. If desired, water-insoluble constituents, for example inorganic pigments, may also be added at this stage to adjust viscosity, generally in the form of aqueous or aqueous/alcoholic dispersions. In addition, to emulsify or disperse the active principles, it can be useful to add emulsifiers and/or solubilisers to the matrix. After its preparation from gel former, cationic polymer and active principle, the matrix optionally is very finely dispersed in an oil phase with intensive shearing in order to produce small particles in the subsequent encapsulation process. It has proved to be particularly advantageous in this regard to heat the matrix to temperatures in the range from 40 to 60° C while the oil phase is cooled to 10 to 20° C. The actual encapsulation, i.e. formation of the membrane by contacting the cationic polymer in the matrix with the anionic polymers, takes place in the third step. To this end, it is advisable to wash the matrix - dispersed in the oil phase - with an aqueous ca. 0.1 to 3 and preferably 0.25 to 0.5% by weight aqueous solution of the anionic polymer, preferably the alginate, at a temperature in the range from 40 to 100 and preferably 50 to 60° C. and, at the same time, to remove the oil phase if present. The resulting aqueous preparations generally have a microcapsule content of 1 to 10% by weight. In some cases, it can be of advantage for the solution of the polymers to contain other ingredients, for example emulsifiers or preservatives. After filtration, microcapsules with a mean diameter of preferably 1 to 3 mm are obtained. It is advisable to sieve the capsules to ensure a uniform size distribution. The microcapsules thus obtained may have any shape within production-related limits, but are preferably substantially spherical.

### ORAL COMPOSITIONS

Typical examples for suitable oral compositions comprising the components (a) and/or (b) and also optionally (c) encompass the following embodiments:
- bakery products;
- candies;
- chewing gums;
- tooth pastes or a mouth washes; and
- pharmaceutical compositions;
- compressed tablets.

Also covered by the present invention is an oral preparation in form of a flavor composition, comprising
(a) hydroxyl-alpha sanshool and
(b) 2-phenylbutenal.

### Bakery products

Bakery products according to the present invention can be made from dough which may be yeast leavened or non-yeast leavened, such as baking powder raised bread. The bakery product is typically selected from artisan bread, bagels, baguette, biscuits, bread loaves, brownies, buns, burger rolls, cakes, cookies, cornmeal, crackers, crepes, cookies such as bar type including toffee, lemon, date, fig and fruit filled cookies, croissants, crumpets, Danish pasty, flat bread, French toast, fruit bread, khari, laminated doughs, moon cakes, muffins, naan, namkeens, nankatais, pan bread, pancakes, pastries including eclairs, cream puff and doughnuts, pies, pita bread, pizza or pizza crust, quick bread, rolls, rye bread, sandwich pouches, scones, sweet dough, sweet rolls, tin bread, toast, tortillas, waffles, wheat bread, whole-wheat bread or yeast raised high moisture specialty breads, including added fiber, low calorie, English muffins. Most typically the bakery product may be yeast raised bread including rolls, sweet rolls, bagels, croissants and pizza crust; flat bread, including naan and pita bread; yeast raised high moisture specialty bread; baking powder raised bread, including biscuits, scones, muffins, cornmeal and quick bread; crumpets; sweet ready-to-eat baked goods including cakes, cheesecake, pies and pastries, or tortillas. The bakery product may be baked, semi-baked, parbaked, pre-baked or non-baked. Also frozen bakery products are included in the definition of bakery product according to the present invention. However, such products may be sufficiently preserved by the freezing rather than by a chemical preservative.

### Candies

According to the present invention the preferred candies are so-called hard-boiled candies. Their bases are usually prepared from a mixture of sugar and other carbohydrates that are kept in an amorphous or glassy condition. This form can be considered a solid syrup of sugars generally having up to about 4.5 % b.w. moisture, based on the weight of the candy base, with about 0.5 to about 2.5 % b.w. being preferred and about 1.0 to about 1.5 % b.w. being most preferred. Such materials normally contain up to 65 % b.w. corn syrup, up to 80 % b.w. sugar and from 0.1 to 5.0 % b.w. water. Generally, the ratio of sugar (or other sweetener suitable for candy formulation) to corn syrup is within the range of about 70:25 to about 45:55 with about 60:40 being preferred. The syrup component generally is prepared from corn syrups high in fructose, but may include other materials. Further ingredients such as flavorings, sweeteners, acidulents, colorants and so forth may also be added.

Hard boiled candy bases may also be prepared from non-fermentable sugars such as sorbitol, mannitol, xylitol, maltitol, hydrogenated starch hydrolysate, hydrogenated corn syrup and mixtures thereof. The candy bases may contain up to about 95% sorbitol, a mixture of sorbitol and mannitol at a ratio of about 9.5 to 0.5 up to about 7.5 to 2.5 and hydrogenated corn syrup up to about 55% of the syrup component.

### Compressed tablets

According to the present invention the oral compositions can represent compressed tablets, comprising the liquid flavor in amounts of typically about 0.1 to about 0.6 % b.w. and preferably about 0.5 % b.w. The amount of hydroxy alpha-sanshool and/or 2-phenyl butenal in amounts of from about 0.01 to about 0.07 % b.w. and preferably about 0.05 % b.w.

### Chewing-gums

Chewing gums typically consist of a water- insoluble vase component, a water-soluble component and additives providing for example a specific flavor.

The ***water-insoluble base,*** which is also known as the "gum base", typically comprises natural or synthetic elastomers, resins, fats and oils, plasticizers, fillers, softeners, dyes and optionally waxes. The base normally makes up 5 to 95% by weight, preferably 10 to 50% by weight and more particularly 20 to 35% by weight of the composition as a whole. In one typical embodiment of the invention, the base consists of 20 to 60% by weight synthetic elastomers, 0 to 30% by weight natural elastomers, 5 to 55% by weight plasticizers, 4 to 35% by weight fillers, 5 to 35% by weight softeners and small amounts of additives, such as dyes, antioxidants and the like, with the proviso that they are soluble in water at best in small quantities.

Suitable synthetic elastomers are, for example, polyisobutylenes with average molecular weights (as measured by GPC) of 10,000 to 100,000 and preferably 50,000 to 80,000, isobutylene/isoprene copolymers ("butyl elastomers"), styrene/butadiene copolymers (styrene:butadiene ratio, for example, 1:3 to 3:1). polyvinyl acetates with average molecular weights (as measured by GPC) of 2,000 to 90,000 and preferably 10,000 to 65,000, polyisoprenes, poly-ethylenes, vinyl acetate/vinyl laurate copolymers and mixtures thereof. Examples of suitable natural elastomers are rubbers, such as for example smoked or liquid latex or guayuls, and natural gums, such as jelutong, lechi caspi, perillo, sorva, massaranduba balata, massaranduba chocolate, nispero, rosindinba, chicle, gutta hang kang and mixtures thereof. The choice of the synthetic and natural elastomers and their mixing ratios essentially depends on whether or not bubbles are to be produced with the chewing gums (bubble gums). Elastomer mixtures containing jelutong, chicle, sorva and massaranduba are preferably used.

In most cases, the elastomers are too hard or lack plasticity for satisfactory processing, so that it has been found to be of advantage to use special plasticizers which, of course, must also satisfy in particular all requirements relating to acceptability as food additives. In this respect, suitable plasticizers are, above all, esters of resin acids, for example esters of lower aliphatic alcohols or polyols with completely or partly hydrogenated, monomeric or oligomeric resin acids. In particular, the methyl, glycerol or pentaerythritol esters or mixtures thereof are used for this purpose. Alternatively, terpene resins, which may be derived from .alpha.-pinene, .beta.-pinene, .delta.-limonene or mixtures thereof, could also be used.

Suitable fillers or texturizers are magnesium or calcium carbonate, ground pumice stone, silicates, especially magnesium or aluminium silicates, clays, aluminium oxides, talcum, titanium dioxide, mono-, di- and tricalcium phosphate and cellulose polymers.

Suitable softeners or emulsifiers are tallow, hydrogenated tallow, hydrogenated or partly hydrogenated vegetable oils, cocoa butter, partial glycerides, lecithin, triacetin and saturated or unsaturated fatty acids containing 6 to 22 and preferably 12 to 18 carbon atoms and mixtures thereof.

Suitable dyes and whiteners are, for example, the FD&C types, plant and fruit extracts permitted for colouring foods and titanium dioxide. The gum bases may also contain waxes or may be wax-free

In addition to the water-insoluble gum base, chewing gum preparations regularly contain a ***water-soluble component*** which is formed, for example, by softeners, sweeteners, fillers, flavors, flavor enhancers, emulsifiers, dyes, acidifiers, antioxidants and the like, with the proviso that the constituents have at least adequate solubility in water. Accordingly, individual constituents may belong both to the water-insoluble phase and to the water-soluble phase, depending on the water solubility of the special representatives. However, combinations may also be used, for example a combination of a water-soluble and a water-insoluble emulsifier, in which case the individual representatives are present in different phases. The water-insoluble component usually makes up 5 to 95% by weight and preferably 20 to 80% by weight of the preparation.

Water-soluble softeners or plasticizers are added to the chewing gum compositions to improve chewability and the chewing feel and are present in the mixtures in quantities of typically 0.5 to 15% by weight. Typical examples are glycerol, lecithin and aqueous solutions of sorbitol, hydrogenated starch hydrolysates or corn sirup.

Fillers are particularly suitable for the production of low-calorie chewing gums and may be selected, for example, from polydextrose, raftilose, raftilin, fructo-oligosaccharides (NutraFlora), palatinose oligosaccharides, guar gum hydrolyzates (Sun Fiber) and dextrins.

The chewing gums may additionally contain auxiliaries and additives which are suitable, for example, for dental care, more particularly for controlling plaque and gingivitis, such as for example chlorhexidine, CPC or triclosan. They may also contain pH adjusters (for example buffer or urea), anti-caries agents (for example phosphates or fluorides), biogenic agents (antibodies, enzymes, caffeine, plant extracts), providing these substances are permitted in foods and do not undesirably interact with one another.

### Toothpastes and mouth washes

Toothpastes or tooth creams are generally understood to be paste-like preparations of water, thickeners, humectants, abrasives or polishes, surfactants, sweeteners, flavorings, deodorizing agents and agents active against oral and dental diseases. In toothpastes according to the invention, any of the usual polishes may be used, such as chalk, dicalcium phosphate, insoluble sodium metaphosphate, aluminium silicates, calcium pyrophosphate, finely particulate synthetic resins, silicas, aluminium oxide and aluminium oxide trihydrate. Particularly suitable polishes for toothpastes according to the invention are finely particulate xerogel silicas, hydrogel silicas, precipitated silicas, aluminium oxide trihydrate and finely particulate.alpha.-alumina, or mixtures of these polishes. Such polishes are preferably used in quantities of from about 15 to 40% by weight of the toothpaste. Preferred humectants used for toothpastes according to the invention include low molecular weight polyethylene glycols, glycerol, sorbitol or mixtures thereof in quantities of up to about 50% by weight of the toothpaste. Among the known thickeners for use with toothpastes according to the invention, particularly preferred are the thickening, finely particulate gel silicas and nonionic hydrocolloids, such as hydroxy ethyl cellulose, hydroxy propyl guar, hydroxy ethyl starch, polyvinyl pyrrolidone, high molecular weight polyethylene glycol and vegetable gums, such as tragacanth, agaragar, carrageen moss, gum arabic and xanthan gum. The desired flavor and aroma for preparations in accordance with the invention may be obtained by adding the components (a) and/or (b) and optionally also (c). It is also advantageous adding caries inhibitors to the oral preparations in the form of, for example, alkali fluorides, alkali monofluorophosphates or alkali salts of organophosphonic acids. In addition, the oral preparations according to the invention may contain other standard auxiliaries, such as dyes, preservatives and opacifiers, for example titanium dioxide. For mouthwashes, the oral compositions according to the invention may readily be combined with aqueous-alcoholic solutions containing different amounts of ethereal oils, emulsifiers, astringent and toning drug extracts, caries-inhibiting additives and flavor correctants.

### ADDITIVES

The oral compositions of the present invention may include additional additives as for examples sweeteners or vitamins, in amounts of from about 0.1 to about 10 % b.w.

### Sweeteners

Suitable sweet-tasting substances, including natural sources of these substances (component e5), such as for example sweet-tasting carbohydrates or sugars (e.g. sucrose (synonymous with saccharose), trehalose, lactose, maltose, melizitose, raffinose, palatinose, lactulose, D-fructose, D-glucose, D-galactose, L-rhamnose, D-sorbose, D-mannose, D-tagatose, D-arabinose, L-arabinose, D-ribose, D-glyceraldehyde, maltodextrin) or vegetable preparations containing predominantly these carbohydrates (e.g. from sugar beet (Beta vulgaris ssp., sugar fractions, sugar syrup, molasses), from sugar cane (Saccharum officinarum ssp., e.g. molasses, sugar syrups), from sugar maple (Acer ssp.), from agave (agave thick juice), synthetic/enzymatic hydrolysates of starch or sucrose (e.g. invert sugar syrup, highly enriched fructose syrups made from corn starch), fruit concentrates (e.g. from apples or pears, apple syrup, pear syrup), sugar alcohols (e.g. erythritol, threitol, arabitol, ribitol, xylitol, sorbitol, mannitol, dulcitol, lactitol), proteins (e.g. miraculin, monellin, thaumatin, curculin, brazzein), sweeteners (magap, sodiumcyclamate, acesulfame K, neohesperidin dihydrochalcone, saccharin sodium salt, aspartame®, superaspartame, neotame, alitame, sucralose, stevioside, rebaudioside, lugduname, carrelame, sucrononate, sucrooctate, monatin, phyllodulcin), certain sweet-tasting amino acids (glycine, D-leucine, D-threonine, D-asparagine, D-phenylalanine, D-tryptophan, L-proline), other sweet-tasting low-molecular substances (e.g. hernandulcin, dihydrochalcone glycosides, glycyrrhizin, glycyrrhetinic acid ammonium salt or other glycyrrhetinic acid derivatives), liquorice extracts (Glycyrrhizza glabra ssp.), Lippia dulcis extracts, Momordica ssp. extracts or individual substances (in particular Momordica grosvenori [Luo Han Guo] and the mogrosides obtained therefrom), Hydrangea dulcis or Stevia ssp. (e.g. Stevia rebaudiana) extracts or individual substances.

### Vitamins

In another embodiment of the present invention the compositions may include vitamins (component e1). Vitamins have diverse biochemical functions. Some have hormone-like functions as regulators of mineral metabolism (e.g., vitamin D), or regulators of cell and tissue growth and differentiation (e.g., some forms of vitamin A). Others function as antioxidants (e.g., vitamin E and sometimes vitamin C). The largest number of vitamins (e.g. B complex vitamins) act as precursors for enzyme cofactors, that help enzymes in their work as catalysts in metabolism. In this role, vitamins may be tightly bound to enzymes as part of prosthetic groups: For example, biotin is part of enzymes involved in making fatty acids. Vitamins may also be less tightly bound to enzyme catalysts as coenzymes, detachable molecules that function to carry chemical groups or electrons between molecules. For example, folic acid carries various forms of carbon group - methyl, formyl, and methylene - in the cell. Although these roles in assisting enzyme-substrate reactions are vitamins' best-known function, the other vitamin functions are equally important. In the course of the present invention suitable vitamins are selected from the group consisting of
- Vitamin A (retinol, retinal, beta carotene),
- Vitamin B₁ (thiamine),
- Vitamin B₂ (riboflavin),
- Vitamin B₃ (niacin, niacinamide),
- Vitamin B₅ (panthothenic acid),
- Vitamin B₆ (pyridoxine, pyridoxamine, paridoxal),
- Vitamin B₇ (biotin),
- Vitamin B₉ (folic acid, folinic acid),
- Vitamin B₁₂ (cyanobalamin, hydoxycobalmin, methylcobalmin),
- Vitamin C (ascorbic acid),
- Vitamin D (cholecalciferol),
- Vitamin E (tocopherols, tocotrienols), and
- Vitamin K (phyolloquinone, menaquinone).

The preferred vitamins are ascorbic acid and tocopherols.

Said vitamins may be present in the food composition in amounts of about 0.1 to about 5 % b.w., and preferably about 0.5 to about 1 % b.w.

### INDUSTRIAL APPLICATION

Another object of the present invention is directed to a method for providing a salivating, moisturizing, tingling, popping and/or watering effect to a flavor to an oral composition by adding
(a) hydroxyl-alpha sanshool and
(b) 2-phenylbutenal
in an amount (a+b) of less than 0.2 % b.w. to said composition. Finally, a last object of the invention covers the use of
(a) hydroxyl-alpha sanshool and
(b) 2-phenylbutenal
as an aroma or flavoring agent for oral compositions.

### EXAMPLES

### EXAMPLE H1

### Manufacture of an extract of Sichuan pepper, rich in Hydroxy-alpha sanshool

Sichuan pepper was extracted by means of supercritical carbon dioxide. The results from GC/MS are reported in **Table 1.** The product was injected 2 % b.w. in TBME and detected by FID or HPLC in the alternative. The components were calculated compared to internal standards (2-nonanol, 1.1 % b.w. added) in ppm.

**Table 1**

| Composition of an extract of Sichuan pepper - only component provided with amounts above 1 % b.w. | | |
|---|---|---|
| **Component** | **Amount [ppm]** | **GC [% b.w.]** |
| Linalyl acetate | 87313,86 | 33,42 |
| Limonene | 59203,96 | 22,66 |
| Linalool | 24801,73 | 9,49 |
| Myrcene | 13276,24 | 5,08 |
| Sabinene | 10444,55 | 4,00 |
| Sabinene hydrate acetate | 9788,37 | 3,75 |
| Hydroxy alpha sanshool | 7855,12 | 3,00 |
| 4-Terpinenol | 7615,59 | 2,91 |
| Eucalyptol | 6134,41 | 2,35 |
| Beta Phellantrene | 6050,00 | 2,32 |
| t-Sabine hydrate | 3651,24 | 1,40 |
| Beta Oximene | 3561,39 | 1,36 |
| Gamma Terpinene | 2929,70 | 1,12 |
| Alpha Terpinylacetate | 2657,43 | 1,02 |

### EXAMPLE H2

### Manufacture of 2-phenylbutenal

51 g sodium acetate was dissolved in a mixture of 100 g water and 100 g ethanol. The solution was kept at 15 °C and 120 g phenyl acetaldehyde and 61.7 acetaldehyde were added portion wise over a period of 60 minutes. The reaction mixture was stirred for about 4 h at about 20 °C and then kept for another 13 hours under reflux. Subsequently, 500 g water was added to the mixture in order to achieve phase separation. The organic layer was extracted using methyl tert-.butyl ether as the extractant. The extracts were combined, washed with water and liberated from the organic solvent providing crude 137 g 2-phenylbutenal. The product was subjected to thin layer distillation at a temperature of about 120 °C and a reduced pressure of about 2 mbar. 74 g distillate was obtained that was further purified by distillation in a packed column. Finally 65.5 g purified 2-phenylbutenal was obtained, consisting of 94.3 % b.w. of the trans- and 3.4 % of the cis-isomer.

### EXAMPLE 1

### Flavor and aroma compositions

The following **Tables 2.1 to 2.5** disclose some flavor and aroma compositions that are useful for flavoring the oral compositions according to the invention. As far as the tables refer to hydroxy-alpha sanshool an extract of sichuan pepper obtained from CO₂ extraction was used.

**Table 2.1**

| Aroma composition A; all amounts in % b.w. | |
|---|---|
| **Composition** | **Amount** |
| Hydroxy-alpha sanshool | 5 |
| 2-Phenylbutenal | 5 |
| Anethol | 9 |
| I-Menthol | 35 |
| Peppermint oil - Piperita type | 26 |
| Peppermint oil - Arvensis type | 27 |
| **Total** | 100 |

**Table 2.2**

| Aroma composition B; all amounts in % b.w. | |
|---|---|
| **Composition** | **Amount** |
| Hydroxy-alpha sanshool | 4 |
| 2-Phenylbutenal | 4 |
| Anethol | 9 |
| I-Menthol | 30 |
| Peppermint oil - Piperita type | 12 |
| Peppermint oil - Arvensis type | 5 |
| I-Carvon | 15 |
| Spearmint oil - Cardiaca type | 15 |
| Spearmint oil - Spicata type | 15 |
| **Total** | 100 |

**Table 2.3**

| Aroma composition C; all amounts in % b.w. | |
|---|---|
| **Composition** | **Amount** |
| Hydroxy-alpha sanshool | 4 |
| 2-Phenylbutenal | 4 |
| Anethol | 9 |
| Peppermint oil - Piperita type | 45 |
| I-Menthol | 2 |
| Peppermint oil - Arvensis type | 3 |
| Spearmint oil - Spicata type | 6 |
| Eugenol | 7 |
| Eucalyptol | 5 |
| Methylsalicylat | 20 |
| **Total** | 100 |

**Table 2.4**

| Aroma composition D; all amounts in % b.w. | |
|---|---|
| **Composition** | **Amount** |
| Hydroxy-alpha sanshool | 6 |
| 2-Phenylbutenal | 6 |
| Anethol | 18 |
| Eucalyptol | 15 |
| Eucalyptus oil | 5 |
| I-Menthol | 44 |
| Peppermint oil - Piperita type | 8 |
| Peppermint oil - Arvensis type | 3 |
| **Total** | 100 |

**Table 2.5**

| Aroma composition E; all amounts in % b.w. | |
|---|---|
| **Composition** | **Amount** |
| Hydroxy-alpha sanshool | 6 |
| 2-Phenylbutenal | 6 |
| Cinnamon aldehyde | 10 |
| Anethol | 9 |
| Eugenol | 2 |
| I-Menthol | 34 |
| Peppermint oil - Piperita type | 10 |
| Peppermint oil - Arvensis type | 15 |
| Spearmint oil - Spicata type | 6 |
| **Total** | 100 |

These aroma compositions may be incorporated into the oral compositions of the invention according to the amounts presented below:
- Tooth pastes: 0.8 - 1.5 % b.w.
- Mouth washes: 0.1 - 0.25 % b.w.
- Mouth wash concentrates: 1 - 4 % b.w.
- Chewing gums: 1 - 1.8 % b.w.
- Candies: 0.1 - 0.4 % b.w.
- Compressed tablet: 0.1 - 0.6% b.w.

### EXAMPLE 2

### Formulation examples

The following **Tables 3.1 to 3.5** provide various examples for oral compositions comprising at least one of the flavor compositions disclosed in Example 2.

**Table 3.1**

| Chewing gum, free of sugar; all amounts in % b.w. | |
|---|---|
| **Composition** | **Amount** |
| Gum base | 30.00 |
| Sorbit, powdered | 40.00 |
| Isomalt, powdered | 9.50 |
| Xylitol | 2.00 |
| Mannit D | 3.00 |
| Aspartame | 0.10 |
| Acesulfam K | 0.10 |
| Emulgum / Plasticizing agent | 0.30 |
| Sorbitol (70% water) | 13.00 |
| Glycerol | 1.00 |
| Aroma composition A | 1.00 |
| **Total** | 100 |

At a dosage of 1 % b.w. the aroma compositions, particularly mixture A3 (which is equivalent to an amount of hydroxy-alpha sanshool and 2-phenylbutenal of 0.1 % b.w.) cause an improved salivating, moisturizing and watering effect. While 2-Phenylbutenal was found to show the effect right from the beginning with decreasing tendency, the effect for hydroxy-alpha sanshool starts later, but remains longer. The effects have also been found more intensive for the combination of the two flavors compared to them taken alone at the same concentration.

**Table 3.2**

| Tooth paste; all amounts in % b.w. | |
|---|---|
| **Composition** | **Amount** |
| Deionised water | 36,39 |
| Glycerol | 20,00 |
| Solbrol M (sodium salt) | 0,15 |
| Sodium monofluor phosphate | 0,76 |
| Saccharin | 0,20 |
| Dicalciumphosphate dihydrate | 36,00 |
| Aerosil 200 | 3,00 |
| Sodium carboxymethyl cellulose | 1,20 |
| Sodium lauryl sulfate | 1,30 |
| Aroma composition B | 1.00 |
| **Total** | 100 |

At a dosage of 1 % b.w. the aroma compositions, particularly mixture B3 (which is equivalent to an amount of hydroxy-alpha sanshool and 2-phenylbutenal of 0.08 % b.w.) cause a boost of flavor and an improved fresh feeling.

**Table 3.3**

| Mouth wash concentrate; all amounts in % b.w. | |
|---|---|
| **Composition** | **Amount** |
| Ethanol 96% | 42,00 |
| Cremophor RH 455 | 5,00 |
| Deionised water | 50,67 |
| Allantoin | 0,20 |
| Sodium saccharin 450 | 0,10 |
| Colour L-Blue 5000 (1% in Wasser) | 0,03 |
| Aroma composition C | 2.00 |
| **Total** | 100 |

**Table 3.4**

| Hard boiled candy, sugar-free; all amounts in % b.w. | |
|---|---|
| **Composition** | **Amount** |
| Water | 2.24 |
| Isomalt | 94.98 |
| Xylitol | 2.40 |
| Sucralose | 0.03 |
| Acesulfame K | 0.050 |
| Citric acid | 0.050 |
| Aroma composition D | 0.25 |
| **Total** | 100 |

**Table 3.5**

| Hard boiled candy; all amounts in % b.w. | |
|---|---|
| **Composition** | **Amount** |
| Water | 2.24 |
| Glucose | 94.98 |
| Glucose syrup | 2.40 |
| Aroma composition E | 0.25 |
| **Total** | 100 |

**Table 3.6**

| Generic compressed tablet formulation; all amounts in % b.w. | |
|---|---|
| **Composition** | **Amount** |
| Bulk Sweetener e.g. Dextrose, Sorbitol | 95 - 98 |
| Excipient/Processing aid | 0.2 - 0.5 |
| Wetting Agent/Disintegrant | 0.2 - 0.5 |
| Intense Sweetener (sugar-free only), e.g. Aspartame | 0.1 - 0.2 |
| Aroma composition, e.g. D1 or D2 | 0.1 - 0.6 |
| Color | As required |
| Acidulant | As required |
| Coating material, e.g. Waxes | As required |
| **Total** | 100 |

## Claims

1. An oral composition comprising
(a) hydroxyl-alpha sanshool and
(b) 2-phenylbutenal,
and optionally
(c) additional flavour or aroma compounds,
on condition that the total amount of components (a+b) is less than 0.2 % b.w.

2. The composition of Claim 1, wherein the total amount of components (a+b) is about 0.0001 to about 0.1 % b.w.

3. The composition of Claim 1, wherein the components (a) and (b) are present in a ratio per weight of about 10:90 to about 90:10.

4. The composition of Claim 1, wherein component (a) represents an extract of Sichuan pepper.

5. The composition of Claim 4, wherein said extract is obtained from Sichuan pepper by extraction using carbon dioxide.

6. The composition of Claim 1, wherein the oral composition is a bakery product.

7. The composition of Claim 1, wherein the oral composition is a chewing gum.

8. The composition of Claim 1, wherein the oral composition is a candy.

9. The composition of Claim 1, wherein the oral composition is a compressed tablet.

10. The composition of Claim 1, wherein the oral composition is a tooth paste or a mouth wash.

11. The composition of Claim 1, wherein the oral composition is a pharmaceutical composition.

12. A flavour composition, comprising
(a) hydroxyl-alpha sanshool and
(b) 2-phenylbutenal.

13. A method for providing a salivating, moisturizing, tingling, popping and/or watering effect to a flavour to an oral composition by adding
(a) hydroxyl-alpha sanshool and
(b) 2-phenylbutenal
in an amount (a+b) of less than 0.2 % b.w. to said composition.

14. The use of
(a) hydroxyl-alpha sanshool and
(b) 2-phenylbutenal
as an aroma or flavoring agent for oral compositions.

## Patentansprüche

1. Orale Zusammensetzung umfassend
(a) Hydroxy-alpha Sanshool und
(b) 2-Phenylbutenal, und optional
(c) zusätzliche Geschmacks- oder Aroma-Verbindungen,
unter der Bedingung, dass die Gesamtmenge der Komponenten (a+b) weniger ist als 0,2 Gew.-%.

2. Zusammensetzung nach Anspruch 1, wobei die Gesamtmenge der Komponenten (a+b) circa 0,0001 bis circa 0,1 Gew.-% ist.

3. Zusammensetzung nach Anspruch 1, wobei die Komponenten (a) und (b) in einem Gewichtsverhältnis von circa 10:90 bis circa 90:10 vorhanden sind.

4. Zusammensetzung nach Anspruch 1, wobei Komponente (a) einen Extrakt von Szechuanpfeffer darstellt.

5. Zusammensetzung nach Anspruch 4, wobei besagter Extrakt aus Szechuanpfeffer durch Extraktion unter Einsatz von Kohlenstoffdioxid erhalten wird.

6. Zusammensetzung nach Anspruch 1, wobei die orale Zusammensetzung eine Backware ist.

7. Zusammensetzung nach Anspruch 1, wobei die orale Zusammensetzung ein Kaugummi ist.

8. Zusammensetzung nach Anspruch 1, wobei die orale Zusammensetzung eine Sü-ßigkeit ist.

9. Zusammensetzung nach Anspruch 1, wobei die orale Zusammensetzung eine gepresste Tablette ist.

10. Zusammensetzung nach Anspruch 1, wobei die orale Zusammensetzung eine Zahnpasta oder ein Mundwasser ist.

11. Zusammensetzung nach Anspruch 1, wobei die orale Zusammensetzung eine pharmazeutische Zusammensetzung ist.

12. Eine Geschmacks-Zusammensetzung, umfassend
(a) Hydroxy-alpha Sanshool und
(b) 2-Phenylbutenal.

13. Verfahren zum Bereitstellen eines speichelfördernden, befeuchtenden, kribbelnden, prickelnden und/oder wässernden Effekts in einer Geschmacks- oder in einer oralen Zusammensetzung durch Zugeben von
(a) Hydroxy-alpha Sanshool und
(b) 2-Phenylbutenal
in einer Menge (a+b) von weniger als 0,2 Gew.-% zu besagter Zusammensetzung.

14. Verwendung von
(a) Hydroxy-alpha Sanshool und
(b) 2-Phenylbutenal
als ein Aroma- oder Geschmacksstoff für orale Zusammensetzungen.

## Revendications

1. Composition orale comprenant
(a) de l'hydroxy-alpha sanshool et
(b) du 2-phénylbuténal, et, en option,
(c) des composés de saveur ou d'arôme supplémentaires,
dans la condition que la quantité totale de composants (a+b) est inférieure à 0,2 % en poids.

2. Composition selon la revendication 1, dans laquelle la quantité totale des composants (a+b) est d'environ 0,0001 à 0,1 % en poids à peu près.

3. Composition selon la revendication 1, dans laquelle les composants (a) et (b) sont présents dans un rapport en poids de 10 : 90 à peu près à 90 : 10 à peu près.

4. Composition selon la revendication 1, dans laquelle le composant (a) représente un extrait de poivre de Sichuan.

5. Composition selon la revendication 4, dans laquelle ledit extrait est obtenu à partir du poivre de Sichuan par extraction en utilisant du dioxyde de carbone.

6. Composition selon la revendication 1, dans laquelle la composition orale est un produit de boulangerie.

7. Composition selon la revendication 1, dans laquelle la composition orale est un chewing-gum.

8. Composition selon la revendication 1, dans laquelle la composition orale est une friandise.

9. Composition selon la revendication 1, dans laquelle la composition orale est un comprimé compressé.

10. Composition selon la revendication 1, dans laquelle la composition orale est un dentifrice ou un bain de bouche.

11. Composition selon la revendication 1, dans laquelle la composition orale est une composition pharmaceutique.

12. Composition de saveur comprenant
(a) de l'hydroxy-alpha sanshool et
(b) du 2-phénylbuténal.

13. Procédé pour fournir à une composition de saveur ou une composition orale un effet salivant, humectant, de fourmillement, de picotement et/ou hydratant en ajoutant, à ladite composition,
(a) de l'hydroxy-alpha sanshool et
(b) du 2-phénylbuténal
en une quantité (a+b) inférieure à 0,2 % en poids.

14. Utilisation de
(a) hydroxy-alpha sanshool et de
(b) 2-phénylbuténal
en tant qu'agent aromatisant ou gustatif pour des compositions orales.
